# FASCICULE DE BREVET EUROPEEN

(11) **EP 0 507 698 B1**
(45) Date de publication et mention de la délivrance du brevet: **24.09.2008**
(21) Numéro de dépôt: 92420065.2
(22) Date de dépôt: 04.03.1992
(51) Int. Cl.: C12N 15/82, C12N 15/29, C12N 5/10, C12N 1/21, C12N 9/10, A01H 5/00

(54) **Promoteurs d'histone**
Histon Promotoren
Histone promotors

(30) Priorité: 05.03.1991 FR 9102873
(43) Date de publication de la demande: 07.10.1992
(73) Titulaire: Bayer CropScience S.A., 69009 Lyon (FR)
(72) Inventeur: Leroux, Bernard, F-69380 Lozanne (FR); Chaubet, Nicole, F-67000 Strasbourg (FR); Freyssinet, Georges, F-69450 Saint Cyr au Mont d'Or (FR); Gigot, Claude, F-67000 Strasbourg (FR)
(74) Mandataire: Monconduit, Hervé

(56) Documents cités:
- WO-A-90/02172
- LEPETIT M. ET AL.: 'Functional studies of plant histone gene promoters in plant protoplasts' ABSTRACTS VIITH IAPTC CONGRESS Juin 1990, page 66
- ROST T.L. ET AL.: 'The distribution and characterization of cycling cells in root tips' J. EXP. BOT. vol. 41, no. C1-4, 1990,
- CROUCH M.L.: 'Debating the responsibilities of plant scientists in the decade of the environment' THE PLANT CELL vol. 2, no. 4, Avril 1990, pages 275 - 277
- CHABOUTE M. ET AL.: 'Genomic organization and nucleotide sequences of two histone H3 and two histone H4 genes of Arabidopsis thaliana' PLANT MOL. BIOL. vol. 8, 1987, pages 179 - 191

## Description

La présente invention concerne de nouveaux promoteurs, de nouveaux gènes chimères les contenant et leur utilisation dans les plantes pour leur conférer une tolérance accrue à des herbicides. Elle concerne également les cellules végétales transformées à l'aide de ces gènes et les plantes transformées régénérées à partir de ces cellules ainsi que les plantes issues de croisements utilisant ces plantes transformées.
On connaît des promoteurs s'exprimant spécifiquement dans les tissus en croissance rapide des plantes comme par exemple le promoteur du gène EF-1α tel que décrit dans la demande de brevet WO 90/02172.

On sait également que les gènes codant pour des protéines histones sont des gènes s'exprimant préférentiellement dans les cellules en division (Chaubet et al., 1986, Plant Mol. Biol. 6: 253-263; Chaboute et al., 1987, Plant Mol. Biol. 8: 179-191; Chaboute et al., 1988, Gene 71: 217-223), et que leurs promoteurs sont capables d'exprimer des protéines rapporteuses telles que la protéine GUS dans des protoplastes en division (Lepetit et al., 1990, Abstracts VIIth IAPTC Congress, Amsterdam, Abstract A2-87: p.66). Le glyphosate, le sulfosate ou la fosamétine sont des herbicides systémiques à large spectre de la famille des phosphonométhylglycines. Ils agissent essentiellement comme inhibiteurs compétitifs de la 5-enol pyruvylshikimate-3-phosphate synthase (EC 2.5.1.19) ou EPSPS vis-à-vis du PEP (phosphoenolpyruvate). Après leur application sur la plante, ils sont véhiculés dans la plante où ils s'accumulent dans les parties à croissance rapide, notamment les apex caulinaires et racinaires, provoquant l'altération jusqu'à la destruction des plantes sensibles.

L'EPSPS plastidiale, cible principale de ces produits est une enzyme de la voie de biosynthèse des acides aminés aromatiques, qui est codée par un ou des gènes nucléaires et synthétisée sous forme d'un précurseur cytoplasmique puis importée dans les plastes où elle s'accumule sous sa forme mature.

La tolérance des plantes au glyphosate et aux produits de la famille est obtenue par introduction stable dans leur génome d'un gène d'EPSPS d'origine végétale ou bactérienne mutée ou non quant aux caractéristiques d'inhibition par le glyphosate du produit de ce gène. Etant donné le mode d'action du glyphosate, il est intéressant de pouvoir exprimer le produit de la traduction de ce gène de façon à permettre son accumulation importante dans les plastes.

Il est connu, par exemple d'après le brevet américain 4,535,060, de conférer à une plante une tolérance à un herbicide du type ci-dessus, en particulier la N-phosphonométhylglycine ou glyphosate, par introduction dans le génome des plantes d'un gène codant pour une EPSPS portant au moins une mutation rendant cette enzyme plus résistante à son inhibiteur compétitif (le glyphosate), après localisation de l'enzyme dans le compartiment plastidial. Ces techniques demandent cependant à être améliorées pour obtenir une plus grande fiabilité dans l'emploi de ces plantes en conditions agronomiques.

Dans la présente description, on entend par "plante" tout organisme multicellulaire différencié capable de photosynthèse et par "cellule végétale" toute cellule issue d'une plante et pouvant constituer des tissus indifférenciés tels que des cals ou des tissus différenciés tels que des embryons ou des parties de plantes des plantes ou des semences.

La présente invention a pour objet la production de plantes transformées ayant une tolérance accrue aux herbicides en général, et particulièrement à ceux de la famille des phosphonométhylglycines, par régénération de cellules transformées à l'aide de nouveaux gènes chimères comportant un gène de tolérance à ces herbicides sous le contrôle d'un promoteur de gène d'histone végétal. L'invention concerne également des plantes transformées plus tolérantes aux herbicides en raison d'une meilleure tolérance des parties à croissance rapide, ainsi que les plantes issues de croisements utilisant ces plantes transformées.

Plus particulièrement l'invention utilise un gène chimère pour conférer aux plantes une tolérance accrue vis à vis d'un herbicide, en particulier d'un herbicide ayant pour cible l'EPSPS, comprenant, dans le sens de la transcription, une zone promotrice, une zone peptide de transit, une séquence codant pour une enzyme de tolérance à un herbicide, en particulier le glyphosate, et une zone signal de polyadénylation, caractérisé en ce que la zone promotrice comprend au moins un promoteur d'un gène d'histone végétal permettant l'expression préférentielle de la protéine de tolérance herbicide dans les zones d'accumulation du glyphosate.

Le gène d'histone provient d'une plante monocotylédone telle que par exemple le blé, le maïs ou le riz ou de préférence d'une plante dicotylédone telle que par exemple la luzerne, le tournesol, le soja, le colza ou de préférence Arabidopsis thaliana.

On utilise de préférence un gène d'histone du type H3 ou de préférence H4.

La zone promotrice du gène chimère selon l'invention peut comprendre en outre avantageusement au moins un fragment d'un promoteur d'un gène s'exprimant naturellement dans les plantes c'est à dire, par exemple une promoteur d'origine virale tel que le promoteur de l'ARN 35S du virus de la mosaïque du choux fleur (CaMv35S) ou d'origine végétale tels que la petite sous unité du gène de la ribulose-1,5-bisphosphate carboxylase(RuBisCO) d'une culture comme par exemple le maïs ou le tournesol.

La zone peptide de transit comprend, dans le sens de la transcription, au moins un peptide de transit d'un gène végétal codant pour une enzyme à localisation plastidiale, une partie de séquence de la partie mature N terminale d'un gène végétal codant pour une enzyme à localisation plastidiale, puis un second peptide de transit d'un gène végétal codant pour une enzyme à localisation plastidiale, de préférence le gène de la RuBisCO.

Les peptides de transit ci-dessus utilisables dans la zone de peptide de transit peuvent être en soi connus, d'origine végétale, par exemple issus de maïs, de tournesol, de pois, de tabac ou autres. Le premier et le second peptide de transit peuvent être identiques, analogues ou différents. Ils peuvent en outre comprendre chacun une ou plusieurs unités peptide de transit.

La partie de séquence de la partie mature N-terminale est issue d'un gène végétal codant pour une enzyme à localisation plastidiale, comme par exemple un gène de maïs, de tournesol, de pois ou autres, l'espèce végétale d'origine pouvant être identique, analogue ou différente de celle dont sont issus respectivement le premier et le second peptide de transit. Par ailleurs la partie de séquence de la partie mature peut comprendre un nombre variable d'acides aminés, généralement de 15 à 40,de préférence de 18 à 33.

La construction de l'ensemble de la zone de peptide de transit peut être de manière en soi connue, notamment par fusion ou tout autre moyen convenable. Cette zone caractéristique a comme rôle de permettre le relargage d'une protéine mature avec une efficacité maximale, de préférence sous forme native.

La séquence codante pour la tolérance herbicide utilisable dans le gène chimère selon l'invention code pour une EPSPS mutée ayant un degré de tolérance au glyphosate. Cette séquence obtenue notamment par mutation du gène EPSPS peut être d'origine bactérienne, par exemple issu de Salmonella typhymurium (et nommée dans la suite "gène AroA"), ou végétale, par exemple de pétunia ou de tomate. Cette séquence peut comprendre une ou plusieurs mutations, par exemple la mutation Pro. 101 en Ser. ou encore les mutations Gly 96 en Ala.

La zone signal de polyadénylation, non traduite en 3' du gène chimère selon l'invention peut être d'origine quelconque, par exemple bactérienne telle que celle du gène de nopaline synthase, ou végétale telle que celle de la petite sous unité de la RuBisCO du maïs ou du tournesol.

Le gène chimère selon l'invention peut comprendre, en plus des parties essentielles ci-dessus, une zone intermédiaire non traduite(linker) entre la zone promotrice et la séquence codante et qui peut être d'origine quelconque, bactérienne, virale ou végétale.

### EXEMPLE 1:CONSTRUCTION D'UN GENE CHIMERE :

On effectue la construction d'un gène chimère selon l'invention à partir des éléments:
1) Promoteur: on isole le promoteur à partir dun clone respectivement d'histone H4 A777 et d'histone H4 A748 d'Arabidopsis thaliana race Strasbourg décrits par Chaboute (thèse de l'Université de Strasbourg 1987). Ces promoteurs, comprenant environ 1kpb entre les sites XhoI utilisés pour l'isoler,sont utilisés tels que ou sous forme dupliquée ou fusionnée avec un promoteur de CaMV35S seul ou double, c'est à dire dont une partie a été dupliquée.
2) Zone de transfert: les deux peptides de transit ainsi que les éléments de protéines matures utilisés proviennent de l'ADNc cloné de la petite sous unité de la RuBisCO de maïs correspondant au gène décrit par Lebrun et al (1987) et de l'ADNc cloné de la petite sous unité de la RuBisCO de tournesol isolé par Waksman et al(1987). Plus précisément, la zone de transfert comprend,dans le sens de la traduction:
   - un peptide de transit de la petite sous unité de la RuBisCO de tournesol,
   - une séquence de 22 acides aminés de la partie mature N terminale de de la petite sous unité de la RuBisCO de maïs,
   - un peptide de transit de la petite sous unité de la RuBisCO de maïs.

   D'autres gènes analogues peuvent contenir respectivement des séquences de 10 à 40 et de préférence de 18 et 33 acides aminés.
   Afin de fournir un élément comparatif, une autre construction a été effectuée contenant, dans le sens de la transcription "promoteur double CaMV35S/ peptide de transit de la PSU de la RuBisCO de tournesol/séquence N-terminale de 22 acides aminés de la PSU de la RuBisCO de maïs/peptide de transit de la PSU de la RuBisCO de maïs/gène AroA/nos"(pRPA-BL 410).
3) Gène de structure: il provient du gène muté (Pro 101 en Ser) de l'EPSPS de Salmonella typhymurium isolé par Stalker et al(1985). Le clone pMG34-2 (fourni par Calgene) a été linéarisé par XbaI puis traité à la nucléase de Vigna radiata. Après recoupure par SmaI les deux extrémités franches ont été ligaturées. Le clone obtenu possède un site NcoI sur l'ATG initiateur ainsi qu'un site SalI à 17pb en aval du codon stop. Ce clone a été nommé pRPA-BL 104.
4) Zone signal de polyadénylation: le fragment provient du gène de la nopaline synthase de pTi37 (Bevan et al,1983). Ce site est contenu dans un fragment MboI de 260pb (Fraley et al, 1983; demande de brevet PCT 84/02913) qui a été traité par la polymérase de Klenow et cloné dans le site Smal de M13 mp 18 pour introduire des sites BamHI et EcoRI respectivement aux extrémités 5' et 3.

Après coupure par BamHI et traitement à la nucléase de Vigna radiata puis coupure EcoRI et traitement par la polymérase de Klenow, le fragment résultant a été introduit dans le vecteur p-BL 20 (demande de brevet français 88/04130) coupé par XbaI et BamHI traités à la polymérase de Klenow. Après recoupure par SalI et SstI,on obtient un fragment d'environ 0,4kpb contenant la séquence nos 3' du côté du site Sal I et la bordure droite de l'ADN-T du côté du site SstI.

### L'assemblage de tous ces éléments a été effectué de la façon suivante:

### Fusion" peptide de transit de la PSU de la RuBisCO de maïs/gène AroA":

Le peptide de transit de la PSU du gène de la RuBisCO de maïs provient d'un fragment EcoRI-SphI de 192pb issu de l'ADNc correspondant au gène de la PSU du gène de la RuBisCO de maïs, décrit par Lebrun et al (1987), possédant un site NcoI recouvrant le codon initiateur de la traduction et un site SphI correspondant au site de clivage du peptide de transit.

Par traitement de l'extrémité SphI avec la polymérase du bactériophage T4 et en la ligaturant avec l'extrémité NcoI, traitée à la polymérase de Klenow, du gène AroA de pRPA-BL 104 recoupé EcoRI, on obtient la fusion traductionelle entre le peptide de transit de maïs et le gène de l'EPSPS bactérienne.

### Fusion peptide de transitde la PSU de la RuBisCO de maïs/séquence de 22 acides aminés de la partie mature du gène de maïs/ gène AroA:

De façon similaire un fragment EcoRI-HindII de 228bp de l'ADNc de la PSU du gène de la RuBisCO de maïs est ligaturé avec l'extrémité NcoI traitée à la polymérase de Klenow du gène AroA de pRPA-BL 104 et recoupé par EcoRI. On obtient une fusion traductionnelle entre le peptide de transit de la PSU de la RuBisCO de maïs,les 22 acides aminés de la partie mature de la PSU de la RuBisCO de maïs et le gène de l'EPSPS bactérienne.

### Peptide de transit de la PSU de la RuBisCO de tournesol:

Le fragment est issu de l'ADNc isolé par Waksman et Freyssinet (1987). Un site SphI a été créé selon la méthode de Zoller et Smith (1984) au site de clivage du peptide de transit. Le peptide de transit de la PSU de la RuBisCO de tournesol ainsi obtenu est un fragment EcoRI-SphI de 171 pb.

### Fusion peptide de transit de la PSU de la RuBisCO de tournesol/séquence de 22 acides aminés de la partie mature de la PSU de la RuBisCO de maïs/ gène AroA:

La construction contenant la fusion peptide de transit de la PSU de la RuBisCO de maïs/séquence de 22 acides aminés de la PSU de la RuBisCO de maïs a été coupée par EcoRI-SphI puis mis en ligation avec le fragment EcoRI-SphI de 171 pb correspondant au peptide de transit de la PSU de la RuBisCO de tournesol. Une construction résultante présente une substitution des fragments EcoRI-SphI et est une fusion traductionnelle "peptide de transit de la PSU de la RuBisCO de tournesol/séquence de 22 acides aminés de la PSU de la RuBisCO de maïs/gène AroA".

Le fragment EcoRI-SalI a été ligaturé avec le fragment SalI-SstI contenantla séquence nos 3' et la bordure droite de l'ADN-T. Le fragment EcoRI-SstI résultant comprenant "peptide de transit de la PSU de la RuBisCO de tournesol/séquence de 22 acides aminés de la PSU de la RuBisCO de maïs/gène AroA/nos 3'/Bordure droite ADN-T" est substitué au fragment EcoRI-SstI contenant la bordure droite de l'ADN-T du plasmide 150 A alpha 2 contenant le promoteur double CaMV. La fusion transcriptionelle "double CaMV/peptide de transit de la PSU de la RuBisCO de tournesol/séquence de 22 acides aminés de la PSU de la RuBisCO de maïs/gène AroA/nos 3' dans le vecteur 150 A alpha 2 a été nommé pRPA-BL 294.

### Fusion"peptide de transit de la PSU de la RuBisCO de tournesol/séquence de 22 acides aminés de la PSU de la RuBisCO de maïs/peptide de transit de la PSU de la RuBisCO de maïs/gène AroA":

La construction ci-dessus est coupée par NcoI-HindIII libérant le gène AroA.Puis elle est mise en ligation avec un fragment NcoI-Hind III de 1,5kpb contenant la fusion"peptide de transit de la PSU de la RuBisCO de maïs/gène AroA". Une construction résultante présente une substitution des fragments NcoI-HindIII et est une fusion traductionelle "peptide de transit de la PSU de la RuBisCO de tournesol/séquence de 22 acides aminés de la PSU de la RuBisCO de maïs/peptide de transit de la PSU de la RuBisCO de maïs/gène AroA".

Le fragment EcoRI-SalI a été ligaturé avec le fragment SalI-SstI contenant la séquence nos 3' et la bordure droite de l'ADN-T. Le fragment EcoRI-SstI résultant comprenant "peptide de transit de la PSU de la RuBisCO de tournesol/séquence de 22 acides aminés de la PSU de la RuBisCO de maïs/peptide de transit de la PSU de RuBisCO de maïs/gène AroA/nos 3'/Bordure droite ADN-T" est substitué au fragment EcoRI-SstI contenant la bordure droite de l'ADN-T du plasmide 150 A alpha 2 contenant le promoteur double CaMV. La fusion transcriptionelle "double CaMV/peptide de transit de la PSU de la RuBisCO de tournesol/séquence de 22 acides aminésde la PSU de la RuBisCO de maïs/peptide de transit de la PSU de la RuBisCO de maïs/gène AroA/nos 3''' dans le vecteur 150 A alpha 2 a été nommé pRPA-BL 410.

Toutes les constructions ont été obtenues par substitution de la zone promotrice double CaMV de la construction pRPA-BL 410 obtenue ci dessus,coupée en EcoRI-HindIII par un fragment HindIII-EcoRI contenant les différents éléments de promoteurs suivants:

### A)Promoteur d'histone simple H4 A748 :

Un fragment Xhol-Xhol d'environ 1 kpb contenant le promoteur d'histone H4 748 d'Arabidopsis thaliana a été ligaturé avec pUC 19 coupé par SalI. Ce clone a ensuite été coupé par XbaI traité à la polymérase de Klénow et EcoRI traité à la polymérase de Klénow puis ligaturé en condition favorisant la recircularisation. Le fragment HindIII-EcoRI contenant le promoteur histone a été substitué au fragment Hind III-EcoRI contenant le promoteur double CaMV de pRPA-BL 410.

Le clone résultant contient "promoteur d'histone H4/peptide de transit de la PSU de la RuBisCO de tournesol/séquence de 22 acides aminés de la PSU de la RuBisCO de maïs/peptide de transit de la PSU de la RuBisCO de maïs/gène AroA/nos" et a été nommé pRPA-BL 498.

### B)Promoteur d'histone simple H4 777

Un fragment Xho1 de 1 kpb environ contenant l'ensemble des régions 5' proximale du gène d'histone H4 777 d'Arabidopsis thaliana a été cloné au site Sal I du plasmide pUC 19 dans une orientation telle que les sites du polylinker de pUC 19 de XbaI à EcoRI soit situés en aval du site d'initiation de la transcription.

### Fusion zone de transit de PSU de RuBisCO de tournesol/AroA

Un fragment EcoRI - Hinc II de l'ADNc de la PSU de RuBisCO de tournesol isolé par Waksman et Freyssinet (1987) codant pour le peptide de transit et 30 acides aminés de la proteine mature a été cloné aux sites EcoRI et Sma I de pUC 18.

Un fragment EcoRI-XbaI a été isolé et inséré en amont du gène AroA de pMG 34-2 (fourni par Calgene) aux sites EcoRI et XbaI. Afin de mettre en phase les 2 régions codantes, l'insert EcoRI-Hind III de ce clone a été inséré dans M13 mp19 aux sites EcoRI et Hind III.

La forme réplicative de l'ADN de ce clone a été linéarisé par XbaI puis traité par la nuclease de Vigna radiata. Après ligation dans des conditions favorisant la recircularisation et transformation, l'ADN de certains clones a été analysé par sequençage. Un des clones obtenu réalise une fusion traductionnelle entre l'élément de PSU de RuBisCO et le fragment AroA, le codon initiateur ATG de ce dernier étant perdu. La séquence de la jonction entre ces deux éléments est la suivante :

### GAC GGGGATCCGGAA

PSU Linker AroA.

Cette fusion comporte donc dans le sens de la traduction: peptide de transit de la PSU de RuBisCO de tournesol/30 acides aminés de la partie mature de la PSU de RuBisCO de tournesol/3 acides aminés correspondant au linker intermédiaire/AroA.

### Construction de pRPA - BL - 240

Le plasmide comprenant le promoteur du gène histone H4 777 décrit ci-dessus a été coupé par XbaI, traité par la polymerase de Klenow et le fragment résultant de la recoupure par Hind III, isolé. Le plasmide contenant la fusion peptide de transit de tournesol/AroA décrite ci-dessus a été coupé par EcoRI, traité par la polymérase de Klenow et le fragment résultant de la recoupure par Hind III isolé. Ces deux fragments ont été mis en ligation en présence de pUC 19 coupé par Hind III. Un des clones résultant présente la structure suivante: promoteur histone/zone codant pour le peptide de transit de la PSU de RuBisCO de tournesol/zone codant pour 30 acides aminés de la partie mature de la PSU de la RuBisCO de tournesol/zone codant pour 3 acides aminés correspondant au linker intermédiaire/zone codant pour AroA.

Ce clone a été coupé par Sal I, traité par la polymérase de Klenow, puis après recoupure par Hind III le fragment isolé a été ligaturé avec le plasmide contenant la zone de polyadénylation coupé par Bam HI, traité par la polymérase de Klenow, puis recoupé par Hind III.

Un des clones résultant présente la structure suivante: promoteur histone/zone codant pour le peptide de transit de la PSU de RuBisCO de tournesol/zone codant pour 30 acides aminés de la partie mature de RuBisCO de tournesol/zone codant pour 3 acides aminés correspondant au linker intermédiaire/zone codant pour AroA/nos3'.

Le plasmide ci-dessus a été linéarisé par Hind III et inséré au site unique Hind III du vecteur pRPA-BL 127 construit de la façon suivante: le vecteur pCGN 1152 possédant un gène de résistance à l'hygromycine sous contrôle du gène de la mannopine synthase et du terminateur du gène Tml (fourni par Calgene) a été digéré par Hind III et religaturé sur lui même, conduisant à la création d'un site unique de clonage Hind III.

Un des clones résultant comporte la fusion transcriptionnelle suivante en partant de la bordure gauche de l'ADN-T du plasmide pRPA-BL 127: promoteur histone/zone de transit de PSU de RuBisCO de tournesol/Aroa/nos3' et a été nommé pRPA-BL-240.

### C) Duplication du promoteur d'histone (= promoteur double histone).

Le promoteur histone H4 dans pUC 19 décrit ci-dessus a été dirigé par NdeI, traité par la polymérase de Klénow, puis recoupé par Hind III. Le fragment éliminé a été substitué par un fragment Hind III - EcoRV purifié par digestion du plasmide portant le promoteur histone H4. Un des clones obtenu a la structure suivante: zone 5' du promoteur H4 de Hind III à Nde I de 530 pb, zone dupliquée correspondant à deux fragments de 330 pb de NdeI à EcoRV directement répétés, zone 3' du promoteur H4 de EcoRV à EcoRI de 140 pb.

Le fragment Hind III-EcoRI contenant le promoteur double histone a été substitué au fragment Hind III-EcoRI contenant le double CaMV de pRPA-BL 410. Le clone résultant contient"double promoteur histone/peptide de transit de la PSU de la RuBisCO de tournesol/séquence de 22 acides aminés de la PSU de la RuBisCO de maïs/peptide de transit de maïs/gène AroA/nos" et a été nommé pRPA-BL 488.

### D)Promoteur hybride double CaMV/histone:

Le promoteur histone H4 A748,dans la cassette HindIII-EcoRI décrite ci-dessus (promoteur histone simple H4 A748), est coupé AccI, traité à la polymérase de Klenow puis coupé EcoRI. Le fragment résultant de 580pb est ligaturé avec le promoteur double CaMV coupé EcoRV-EcoRI. Le clone résultant comprenant la fusion double CaMV partie 3' du promoteur histone est coupé HindIII-EcoRI et substitué au fragment HindIII-EcoRI de pRPA-BL 410 comprenant le double CaMV. La construction résultante comprend"promoteur double CaMV/partie 3' promoteur histone/peptide de transit de la PSU de la RuBisCO de tournesol/séquence de 22 acides aminés de la PSU de la RuBisCO de maïs/peptide de transit de la PSU de la RuBisCO de maïs/gène AroA/nos" et a été nommé pRPA-BL 502.

### EXEMPLE 2: RESISTANCE DES PLANTES TRANSFORMEES

### 1°Transformation:

Le vecteur est introduit dans la souche non oncogène d'Agrobacterium EHA 101 (Hood et al,1987) porteuse du cosmide pTVK 291 (Komari et al,1986). La technique de transformation est basée sur la procédure de Horsh et al (1985).

### 2°Régénération:

La régénération du tabac PBD6 (provenance SEITA France) à partir d'explants foliaires est réalisée sur un milieu de base Murashige et Skoog (MS) comprenant 30g/l de saccharose ainsi que 200ug/ml de kanamycine. Les explants foliaires sont prélevés sur des plants en serre ou in vitro et transformés selon la technique des disques foliaires (Science 1985, Vol 227, p.1229-1231) en trois étapes successives: la première comprend l'induction des pousses sur un milieu MS additionné de 30g de saccharose contenant 0.05mg d'acide naphtylacétique (ANA) et 2 mg/l de benzylaminopurine (BAP) pendant 15 jours. Les pousses formées au cours de cette étape sont ensuite développées par culture sur un milieu MS additionné de 30 g/l de saccharose mais ne contenant pas d'hormone, pendant 10 jours. Puis on prélève des pousses développées et on les cultive sur un milieu d'enracinement MS dilué au demi à teneur moitié en sels, vitamines et sucres et ne contenant pas d'hormone. Au bout d'environ 15 jours,les pousses enracinées sont passées en terre.

### 3°Mesure de la tolérance au glyphosate:

Les meilleurs plantes issues de la régénération des tabacs transformés sont sélectionnées en serre après traitement des plantes, au stade 5 feuilles par pulvérisation avec une suspension aqueuse de glyphosate à une dose correspondant à 0,6kg/ha de matière active. Les plantes sélectionnées ont été autofécondées et les graines semées sur milieu MS additionné de 30g/l de sucrose et 200microg/ml de kanamycine. Les graines issues de plantes présentant un ratio de ségrégation de 3/4 de plantes résistantes- 1/4 de plantes sensibles ont été conservées. Une analyse par hybridation moléculaire a permis de déterminer les plantes n'ayant intégré qu'une copie de l'ADN-T. Par croisement et sélection, ces plantes ont été amenées à l'état hétérozygotes ou homozygotes. Ces plantes ont été semées en conditions naturelles de plein champ et pulvérisées au stade 5 feuilles avec une dose de 0,8kg/ha de glyphosate (Round Up). Les résultats correspondants à l'observation d'indices de phytotoxicité relevés au bout de 30 jours après le traitement. Dans ces conditions on constate que les plants transformés par les constructions présentent une tolérance acceptable (pRPA-BL 240 et 410) voire bonne (pRPA-BL 488 et 502) alors que les plants témoins sont complètement détruits.

Ces résultats montrent clairement l'amélioration apportéé par utilisation d'un gène chimère selon l'invention pour un même gène codant pour la tolérance au glyphosate.

Les plantes transformées selon l'invention peuvent être utilisées comme parents pour l'obtention de lignées et d'hybrides ayant une tolérance accrue au glyphosate.

### EXEMPLE 3

Des colzas de printemps, cultivar Westar, résistants au glyphosate, ont été obtenus en utilisant la technique de BOULTER et al., 1990 (Plant Science, 70: 91-99) avec les constructions pRPA-BL 488 (dH-At-PTO-aroA-nos), pRPA-BL 502 (CaMV/H-At-PTO-aroA-nos). Ces plantes ont résisté à un traitement en serre au glyphosate à 400g m.a./ha, traitement qui détruit les plantes non transgéniques.

## Revendications

1. Plante transformée de tolérance herbicide améliorée, obtenue à partir d'une cellule végétale transformée comprenant un gène chimère comprenant, dans le sens de la transcription, une zone promotrice, une zone peptide de transit, une séquence codant une protéine de tolérance vis-à-vis d'un herbicide s'accumulant dans les tissus à croissance rapide des plantes, et une zone signal de polyadénylation, **caractérisé en ce que** la zone promotrice comprend au moins un promoteur d'un gène d'histone végétal permettant l'expression de la protéine de tolérance vis-à-vis d'un herbicide dans les zones d'accumulation dudit herbicide, ladite tolérance herbicide des plantes transformées étant améliorée par rapport à des plantes ne comprenant pas ledit gène chimère.

2. Plante transformée selon la revendication 1, **caractérisée en ce que** ledit gène d'histone végétal provient d'une plante dicotylédone

3. Plante transformée selon la revendication 1, **caractérisée en ce que** ledit gène d'histone végétal provient d*'Arabidopsis*

4. Plante transformée selon la revendication 1, **caractérisée en ce que** ledit gène d'histone végétal provient d'une plante monocotylédone

5. Plante transformée selon l'une des revendications 1 à 4, **caractérisée en ce que** ledit gène d'histone végétal est un gène d'histone H4

6. Plante transformée selon l'une des revendications 1 à 5, **caractérisée en ce que** ledit promoteur de gène d'histone végétal est dupliqué

7. Plante transformée selon l'une des revendications 1 à 5, **caractérisée en ce que** ledit promoteur de gène d'histone végétal est associé à un promoteur d'un gène différent pouvant s'exprimer naturellement dans les plantes

8. Plante transformée selon la revendication 7, **caractérisée en ce que** ledit promoteur d'un gène différent est le promoteur du gène codant l'ARN 35S du virus de la mosaïque du Chou-fleur

9. Plante transformée selon l'une des revendications 1 à 8, **caractérisée en ce que** l'herbicide est un herbicide de la famille des phosphonométhylglycines

10. Plante transformée selon la revendication 9, **caractérisée en ce que** l'herbicide de la famille des phosphonométhylglycines est le glyphosate

11. Plante transformée selon l'une des revendications 1 à 10, **caractérisée en ce que** la protéine de tolérance vis-à-vis d'un herbicide est une enzyme EPSPS

12. Plante transformée selon la revendication 11, **caractérisée en ce que** l'enzyme EPSPS est une enzyme EPSPS mutée

13. Plante transformée selon l'une des revendications 11 ou 12, **caractérisée en ce que** l'EPSPS est d'origine bactérienne

14. Plante transformée selon l'une des revendications 11 ou 12, **caractérisée en ce que** l'EPSPS est d'origine végétale

15. Plante transformée selon l'une des revendications 1 à 14, **caractérisée en ce que** la zone peptide de transit est une séquence codant pour un peptide de transit comprenant, dans le sens de la transcription, au moins un peptide de transit d'un gène végétal codant pour une enzyme à localisation plastidiale, une partie de séquence de la partie mature N-terminale d'un gène végétal codant pour une enzyme à localisation plastidiale, puis un second peptide de transit d'un gène végétal codant pour une enzyme à localisation plastidiale.

## Claims

1. Transformed plant with improved herbicidal tolerance, obtained from a transformed plant cell comprising a chimeric gene comprising, in the direction of transcription, a promoter region, a transit peptide region, a sequence encoding a protein for tolerance with respect to a herbicide that accumulates in rapidly growing tissues of plants, and a polyadenylation signal region, **characterized in that** the promoter region comprises at least one promoter of a plant histone gene which allows the expression of the protein for tolerance with respect to a herbicide in the areas where said herbicide accumulates, said herbicidal tolerance of the transformed plants being improved compared with plants which do not comprise said chimeric gene.

2. Transformed plant according to Claim 1, **characterized in that** said plant histone gene originates from a dicotyledonous plant.

3. Transformed plant according to Claim 1, **characterized in that** said plant histone gene originates from *Arabidopsis.*

4. Transformed plant according to Claim 1, **characterized in that** said plant histone gene originates from a monocotyledonous plant.

5. Transformed plant according to one of Claims 1 to 4, **characterized in that** said plant histone gene is a histone H4 gene.

6. Transformed plant according to one of Claims 1 to 5, **characterized in that** said plant histone gene promoter is duplicated.

7. Transformed plant according to one of Claims 1 to 5, **characterized in that** said plant histone gene promoter is associated with a promoter of a different gene that can be expressed naturally in plants.

8. Transformed plant according to Claim 7, **characterized in that** said promoter of a different gene is the promoter of the gene encoding the 35S RNA of the cauliflower mosaic virus.

9. Transformed plant according to one of Claims 1 to 8, **characterized in that** the herbicide is a herbicide of the phosphonomethylglycine family.

10. Transformed plant according to Claim 9, **characterized in that** the herbicide of the phosphonomethylglycine family is glyphosate.

11. Transformed plant according to one of Claims 1 to 10, **characterized in that** the protein for tolerance with respect to a herbicide is an EPSPS enzyme.

12. Transformed plant according to Claim 11, **characterized in that** the EPSPS enzyme is a mutated EPSPS enzyme.

13. Transformed plant according to either of Claims 11 and 12, **characterized in that** the EPSPS is of bacterial origin.

14. Transformed plant according to either of Claims 11 and 12, **characterized in that** the EPSPS is of plant origin.

15. Transformed plant according to one of Claims 1 to 14, **characterized in that** the transit peptide region is a sequence encoding a transit peptide comprising, in the direction of transcription, at least one transit peptide of a plant gene encoding an enzyme located in plastics, a portion of sequence of the mature N-terminal portion of a plant gene encoding an enzyme located in plastics, and then a second transit peptide of a plant gene encoding an enzyme located in plastics.

## Patentansprüche

1. Transformierte Pflanze mit verbesserter Herbizidtoleranz, die ausgehend von einer transformierten Pflanzenzelle erhalten wurde, die ein chimäres Gen enthält, das in Transkriptionsrichtung eine Promoterregion, eine Transitpeptidregion, eine Kodiersequenz für ein Protein für Toleranz gegenüber einem Herbizid, das sich im ganzen Gewebe mit raschem Wachstum akkumuliert, und eine Polyadenylierungssignalregion enthält, **dadurch gekennzeichnet, daß** die Promoterregion mindestens einen Promoter eines pflanzlichen Histongens umfaßt, der die Expression des Proteins für Toleranz gegenüber einem Herbizid in den Akkumulationsregionen dieses Herbizids gestattet, wobei die Herbizidtoleranz der transformierten Pflanzen im Vergleich zu Pflanzen, die dieses chimäre Gen nicht umfassen, verbessert ist.

2. Transformierte Pflanze nach Anspruch 1, **dadurch gekennzeichnet, daß** das pflanzliche Histongen aus einer zweikeimblättrigen Pflanze stammt.

3. Transformierte Pflanze nach Anspruch 1, **dadurch gekennzeichnet, daß** das pflanzliche Histongen aus einer *Arabidopsis*-Pflanze stammt.

4. Transformierte Pflanze nach Anspruch 1, **dadurch gekennzeichnet, daß** das pflanzliche Histongen aus einer einkeimblättrigen Pflanze stammt.

5. Transformierte Pflanze nach einem der Ansprüche 1 bis 4, **dadurch gekennzeichnet, daß** es sich bei dem pflanzlichem Histongen um ein H4-Histongen handelt.

6. Transformierte Pflanze nach einem der Ansprüche 1 bis 5, **dadurch gekennzeichnet, daß** der Promoter des pflanzlichen Histongens verdoppelt ist.

7. Transformierte Pflanze nach einem der Ansprüche 1 bis 5, **dadurch gekennzeichnet, daß** der Promoter des pflanzlichen Histongens mit einem Promoter eines anderen Gens, der natürlich in Pflanzen exprimiert werden kann, verbunden ist.

8. Transformierte Pflanze nach Anspruch 7, **dadurch gekennzeichnet, daß** es sich bei dem Promoter eines anderen Gens um den Promoter des Gens, das für die 35S-RNA des Blumenkohlmosaikvirus kodiert, handelt.

9. Transformierte Pflanze nach einem der Ansprüche 1 bis 8, **dadurch gekennzeichnet, daß** es sich bei dem Herbizid um ein Herbizid der Familie der Phosphonomethylglycine handelt.

10. Transformierte Pflanze nach Anspruch 9, **dadurch gekennzeichnet, daß** es sich bei dem Herbizid der Familie der Phosphonomethylglycine um Glyphosat handelt.

11. Transformierte Pflanze nach einem der Ansprüche 1 bis 10, **dadurch gekennzeichnet, daß** es sich bei dem Protein für Toleranz gegenüber einem Herbizid um ein EPSPS-Enzym handelt.

12. Transformierte Pflanze nach Anspruch 11, **dadurch gekennzeichnet, daß** es sich bei dem EPSPS-Enzym um ein mutiertes EPSPS-Enzym handelt.

13. Transformierte Pflanze nach einem der Ansprüche 11 oder 12, **dadurch gekennzeichnet, daß** die EPSPS bakteriellen Ursprungs ist.

14. Transformierte Pflanze nach einem der Ansprüche 11 oder 12, **dadurch gekennzeichnet, daß** die EPSPS, pflanzlichen Ursprungs ist.

15. Transformierte Pflanze nach einem der Ansprüche 1 bis 14, **dadurch gekennzeichnet, daß** es sich bei der Transitpeptidregion um eine Sequenz handelt, die für ein Transitpeptid kodiert und die in Transkriptionsrichtung folgendes umfaßt: mindestens ein Transitpeptid eines pflanzlichen Gens, das für ein Enzym mit Lokalisierung im Plastiden kodiert, einen Sequenzabschnitt des reifen N-terminalen Teils eines pflanzlichen Gens, das für ein Enzym mit Lokalisation im Plastiden kodiert, und anschließend ein zweites Transitpeptid eines pflanzlichen Gens, das für ein Enzym mit Lokalisation im Plastiden kodiert.
